# EUROPÄISCHE PATENTSCHRIFT

(11) **EP 2 347 249 B1**
(45) Veröffentlichungstag und Bekanntmachung des Hinweises auf die Patenterteilung: **26.02.2014**
(21) Anmeldenummer: 09748934.8
(22) Anmeldetag: 12.10.2009
(51) Int. Cl.: G01N 27/22, G01N 33/36

(54) **Vorrichtung und Verfahren zur kapazitiven Untersuchung eines länglichen textilen Prüfgutes**
Device and method for capacitively inspecting an elongated textile test material
Dispositif et procédé d'inspection capacitive d'un échantillon textile de forme allongée

(30) Priorität: 16.10.2008 CH 16452008
(43) Veröffentlichungstag der Anmeldung: 27.07.2011
(73) Patentinhaber: Uster Technologies AG, 8610 Uster (CH)
(72) Erfinder: GEHRIG, Reto, 8406 Winterthur (CH)
(86) Internationale Anmeldenummer: PCT/CH2009/000328
(87) Internationale Veröffentlichungsnummer: WO 2010/043064

(56) Entgegenhaltungen:
- EP-A1- 0 924 513
- DE-A1- 2 510 644
- US-A- 3 864 626
- US-A- 3 986 108
- US-B1- 6 369 588
- Hewlett-Packard XP002563591 Gefunden im Internet: URL:http://www.hparchive.com/Catalogs/HP-C atalog-1966-1967.pdf> [gefunden am 2010-01-15]
- "IEEE Guide for Measurement of Environmental Sensitivities of Standard Frequency Generators (Revision of IEEE Std 1193 - 1994);IEEE Std 1193-2003 (Revision of IEEE Std 1193-1994) ED - Anonymous", IEEE STANDARD; [IEEE STANDARD], IEEE, PISCATAWAY, NJ, USA, 1 January 2004 (2004-01-01), pages _1-74, XP017603657, ISBN: 978-0-7381-3711-7
- KOENIG R: "ZUR BESCHR[NKUNG DES ANSPRUCHSINHALTS DURCHBAR-DERIVATE", MITTEILUNGEN DER DEUTSCHEN PATENTANWAELTE, HEYMANN, KOLN, DE, 1 January 1997 (1997-01-01), page 62, XP001248632, ISSN: 0026-6884

## Beschreibung

### FACHGEBIET

Die vorliegende Erfindung liegt auf dem Gebiet der kapazitiven Prüfung von länglichen, textilen Gebilden wie Kardenband, Vorgarn, Garn oder Gewebe. Somit betrifft die Erfindung eine Vorrichtung zur kapazitiven Untersuchung eines bewegten länglichen Prüfgutes, gemäss dem Oberbegriff des Anspruchs 1. Eine derartige Untersuchung kann bspw. die Detektion von Fremdstoffen, das Erkennen von Änderungen der Masse pro Längeneinheit und/oder die Messung von Feuchtigkeit im Prüfgut zum Ziel haben. Die Erfindung kann bspw. im Produktionsprozess (online) in Garnreinigern auf Spinn- oder Spulmaschinen oder in der Laborprüfung (offline) in Garnprüfgeräten eingesetzt werden.

### STAND DER TECHNIK

Es ist eine Vielzahl verschiedenartiger Vorrichtungen zur Untersuchung oder Prüfung von länglichem textilem Prüfgut wie bspw. Kardenband, Vorgarn, Garn oder Gewebe bekannt. Sie lassen sich nach ihrer Anwendung in die beiden Klassen Laborprüfung (offline) und Prüfung während des Produktionsprozesses (online) einteilen. Die Vorrichtungen verwenden verschiedene bekannte Sensorprinzipien; hier interessiert besonders das kapazitive Messprinzip. Bei diesem ist ein Messkondensator typischerweise als ebener Plattenkondensator ausgeführt und weist eine Durchgangsöffnung für das Prüfgut auf. Der Messkondensator ist Teil eines LC-Oszillators, so dass bei Anregung des LC-Oszillators eine elektrische Wechselspannung am Messkondensator anliegt. Die Durchgangsöffnung wird dadurch mit einem elektrischen Wechselfeld beaufschlagt. Das Prüfgut wird durch den Plattenkondensator hindurch bewegt und dem Wechselfeld ausgesetzt. Es wird ein elektrisches Ausgangssignal des Plattenkondensators detektiert. In einer Auswerteschaltung werden aus dem Ausgangssignal dielektrische Eigenschaften des Prüfgutes ermittelt. Aus den dielektrischen Eigenschaften werden Änderungen von Parametern des Prüfgutes wie Masse pro Längeneinheit und/oder Materialzusammensetzung bestimmt. Ein kapazitiver Garn- oder Bandsensor ist z. B. in der GB-638,365 A beschrieben.

Um genaue, von äusseren Einflüssen wie Lufttemperatur oder Luftfeuchtigkeit nicht beeinflusste Messungen durchführen zu können, wird häufig eine Kompensationsmethode angewendet. Zu diesem Zweck beinhaltet die Vorrichtung nebst dem eigentlichen Messkondensator einen Referenzkondensator. Dieser kann durch Zufügen einer dritten, parallel zu den beiden Messkondensatorplatten angeordneten Kondensatorplatte gebildet werden, wobei die drei Kondensatorplatten zu einer kapazitiven Messschaltung zusammen geschaltet werden. Beispiele für Messschaltungen und geeignete Auswerteschaltungen für deren Ausgangssignale finden sich in den Schriften EP-0'924'513 A1, WO-2006/105676 A1 und WO-2007/115416 A1.

Aus der US-4,843,879 A ist ein Gerät für die kapazitive Qualitätskontrolle von textilen Fäden bekannt. Es beinhaltet eine Doppelkondensatoranordnung mit einem Messkondensator und einem Referenzkondensator. Die Doppelkondensatoranordnung ist in einem elektrischen Schaltkreis eingebaut. Der Schaltkreis beinhaltet einen Oszillator zum Anlegen zweier Wechselspannungen mit entgegengesetzten Phasen an die beiden äusseren Kondensatorelektroden der Doppelkondensatoranordnung. In jedem Ast zwischen dem Oszillator und der jeweiligen Elektrode befinden sich ein Signalverstärker und ein Abgleichkondensator, mit welchem das Ausgangssignal der Doppelkondensatoranordnung ohne Prüfgut auf den Wert Null abgeglichen werden kann.

Die WO-2006/069720 A2 offenbart eine Messvorrichtung und ein Messverfahren zur kapazitiven Erkennung von Fremdkörpern in einem Produkt, insbesondere in Tabak, Baumwolle oder einem anderen Faserprodukt. Eine Hochfrequenzerzeugungseinrichtung erzeugt eine Hochfrequenzwelle, die an den Messkondensator angelegt wird. Die Spannungsamplitude der erzeugten Hochfrequenzwelle wird mittels einer Regeleinrichtung konstant gehalten.

Aus der EP-0'922'963 A2 ist eine Auswerteschaltung zur Ermittlung komplexer Impedanzen bekannt. Eine Spannungsquelle erzeugt zwei Wechselspannungssignale, von denen eines an die auszumessende Impedanz angelegt wird. Das Ausgangssignal der Impedanz wird mit dem anderen Wechselspannungssignal, welches jeweils um 0° oder 90° phasenverschoben ist, dergestalt mit dem kombiniert, dass aus den sich jeweils ergebenden Ausgangssignalen der Realteil und der Imaginärteil der Impedanz ermittelbar ist. Die Spannungsquelle umfasst einen Frequenzgenerator zur Erzeugung von Digitalsignalen, einen 1:2-Frequenzteiler und ein D-Flip-Flop zur Erzeugung zweier um 90° phasenverschobener Wechselspannungssignale. Die Phasenverschiebung bleibt bei Variation der Frequenz erhalten.

Die JP-2002-005971 A zeigt eine Vorrichtung zur kapazitiven Unterscheidung verschiedener Flüssigkeiten wie destilliertem Wasser und Hahnenwasser und zur Detektion von nichtmetallischen Fremdstoffen in Flüssigkeiten. Zu diesem Zweck wird ein Schaltkreis, in dem sich die Probe befindet, breitbandig abgetastet und die Dispersion ermittelt. Als Signalgenerator für die Abtastung dient ein direkter digitaler Synthesizer (direct digital synthesizer, DDS).

Bei den aus dem Stand der Technik bekannten kapazitiven Sensoren ist der Messkondensator bzw. die Messschaltung ein Teil eines LC-Oszillators. Somit beeinflusst der Messkondensator Parameter der an ihm anliegenden Wechselspannung wie z. B. ihre Frequenz, Phase und Amplitude. Andererseits hat ein LC-Oszillator üblicherweise eine einzige Resonanzfrequenz, oder zumindest eine sehr beschränkte Anzahl von diskreten Resonanzfrequenzen, auf denen er betrieben werden kann. Die Resonanzfrequenz und die Phase können nur mit grossem Aufwand geändert oder abgestimmt werden.

### DARSTELLUNG DER ERFINDUNG

Aufgabe der Erfindung ist es, eine Vorrichtung und ein Verfahren zur kapazitiven Untersuchung eines bewegten länglichen textilen Prüfgutes wie Kardenband, Vorgarn, Garn oder Gewebe zu schaffen, mit denen das Prüfgut besser und vollständiger charakterisiert werden kann. Insbesondere sollen Parameter des textilen Prüfgutes wie Vorhandensein, Art und Anteile von Fremdstoffen, Änderungen der Masse pro Längeneinheit oder Feuchtigkeit zuverlässig bestimmt und voneinander unterschieden werden können.

Diese und andere Aufgaben werden durch die erfindungsgemässen Vorrichtungen und die erfindungsgemässen Verfahren, wie sie in den unabhängigen Patentansprüchen definiert sind, gelöst. Vorteilhafte Ausführungsformen sind in den abhängigen Patentansprüchen angegeben.

Die Erfindung beruht auf der Idee, die Kondensatoranordnung von einem sie treibenden Wechselsignalgenerator derart abzukoppeln, dass sie Parameter des vom Wechselsignalgenerator erzeugten elektrischen Wechselsignals, insbesondere eine Grundfrequenz und eine Signalform des Wechselsignals, nicht wesentlich beeinflusst. Der Wechselsignalgenerator und die Kondensatoranordnung sind somit voneinander unabhängige Komponenten. Insbesondere ist die Kondensatoranordnung nicht mehr Teil des Wechselsignalgenerators. Mit dem Wechselsignalgenerator kann also ein Wechselsignal mit fast beliebigen Parametern, insbesondere Grundfrequenz und Signalform, erzeugt und an die Kondensatoranordnung angelegt werden.

Unter dem Begriff "Kondensatoranordnung" ist ein Kondensator zu verstehen, mit zwei voneinander beabstandeten Platten, zwischen denen sich Luft befindet und zwischen die ein zu untersuchendes bewegtes längliches textiles Prüfgut einführbar ist. Unter dem Begriff "elektrisches Wechselsignal" wird in dieser Schrift ein elektrisches Spannungs- oder Stromsignal mit mindestens einem sich zeitlich ändernden, vorzugsweise periodischen Anteil (AC-Anteil) verstanden, dem zusätzlich ein zeitlich im Wesentlichen konstanter Anteil (DC-Anteil, Offset) überlagert sein kann. Der periodische Anteil weist eine bestimmte Grundfrequenz und eine bestimmte Signalform auf. Dabei ist die "Grundfrequenz" die tiefste Frequenz, die im Frequenzspektrum des Wechselsignals auftritt. Beispiele für Signalformen sind Sinus, Dreieck, Sägezahn, Rechteck etc., wobei sich das entsprechende Grundmuster mit der Grundfrequenz periodisch wiederholt. Die Forderung, wonach die Kondensatoranordnung Parameter des elektrischen Wechselsignals "nicht wesentlich" beeinflusst, verlangt, dass die Kondensatoranordnung jedenfalls nicht frequenzbestimmender Teil eines Messschwingkreises ist. Resonante Komponenten des Wechselsignals, die von der Kondensatoranordnung verursacht sind, sind nicht ausgeschlossen.

Dementsprechend beinhaltet die erfindungsgemäße Vorrichtung mindestens einen Wechselsignalgenerator zum Anlegen eines elektrischen Wechselsignals mit einer bestimmten Grundfrequenz und einer bestimmten Signalform an die Kondensatoranordnung. Die Kondensatoranordnung ist vom mindestens einen Wechselsignalgenerator derart abgekoppelt, dass sie die Grundfrequenz und die Signalform des angelegten Wechselsignals nicht wesentlich beeinflusst. Die Vorrichtung beinhaltet ferner Detektionsmittel zur Detektion mindestens einer elektrischen Messgrösse des an der Kondensatoranordnung abgegriffenen Wechselsignals. Der mindestens eine Wechselsignalgenerator ist dazu eingerichtet, die Grundfrequenz und/oder die Signal form des angelegten Wechselsignals zu verändern.

Der mindestens eine Wechselsignalgenerator ist vorzugsweise derart eingerichtet, dass zusätzlich eine Phasenlage und/oder eine Amplitude des von ihm erzeugten Wechselsignals vorgebbar sind. Zur Abkopplung ist dem mindestens einen Wechselsignalgenerator vorzugsweise mindestens ein Verstärker zur Verstärkung des vom Wechselsignalgenerator erzeugten Wechselsignals nachgeschaltet. Auch ein Filter zur Filterung des vom Wechselsignalgenerator erzeugten Wechselsignals kann dem mindestens einen Wechselsignalgenerator nachgeschaltet sein.

Der mindestens eine Wechselsignalgenerator ist vorzugsweise aus der folgenden Menge ausgewählt: RC-Oszillator, LC-Oszillator, Quarz-Oszillator, Oszillator mit Keramikresonator, Oszillator mit SAW-Komponente (akustische Oberflächenwellen, surface acoustic waves, SAW), Oszillator mit Logikbausteinen, Synthesizer, Phasenregelschleife (phase-locked loop, PLL), Pulsweitenmodulator (pulse-width modulator, PWM), Kippstufe.

In einer bevorzugten Ausführungsform wird als Wechselsignalgenerator ein Synthesizer verwendet. Unter dem Begriff "Synthesizer" wird in dieser Schrift eine mixed-signal (digitale und analoge) elektronische Vorrichtung zur Erzeugung von analogen elektrischen Wechselspannungssignalen verstanden. Mit besonderem Vorteil werden in der erfindungsgemässen Vorrichtung direkte digitale Synthesizer (direct digital synthesizer, DDS) eingesetzt. Ein DDS ist ein elektronischer Baustein, der im Prinzip analoge Signale beliebiger Signalform, Frequenz und/oder Phasenlage erzeugen kann, wobei einzelite Typen gewissen Beschränkungen unterliegen können. Er hat eine digitale Hardware, die mit einer festen Frequenz (Clockfrequenz) betrieben wird. In einem Rechenspeicher (programmable read-only memory, PROM) des DDS sind digitale Werte einer ganzen oder halben Periode eines periodischen Signals, bspw. eines Sinussignals, als Tabelle abgelegt. Diese Stützstellen werden bei der Signalerzeugung abgerufen. Dabei können gewisse Stützstellen ausgelassen oder verdoppelt werden, so dass sich beliebige Frequenzen erzeugen lassen.

In einer bevorzugten Ausführungsform sind mehrere Wechselsignalgeneratoren vorhanden, und Steuermittel zur Ansteuerung der Wechselsignalgeneratoren sind derart ausgebildet, dass Phasen von Wechselspannungssignalen verschiedener Wechselsignalgeneratoren um vorgegebene Phasendifferenzen gegeneinander phasenverschiebbar sind.

Die erfindungsgemäße Vorrichtung kann einen Referenzkondensator beinhalten, welcher in Serie zur auszumessenden Kapazität geschaltet ist.

Die erfindungsgemäße Vorrichtung dient zur kapazitiven Untersuchung eines bewegten länglichen textilen Prüfgutes wie Kardenband, Vorgarn, Garn oder Gewebe, wobei das bewegte Prüfgut die auszumessende Kapazität beeinflusst.

Gegenstand der vorliegenden Erfindung ist also eine Vorrichtung zur kapazitiven Untersuchung eines bewegten länglichen, vorzugsweise textilen Prüfgutes wie Kardenband, Vorgarn, Garn oder Gewebe. Sie beinhaltet eine kapazitive Messschaltung mit einem Messkondensator zur Aufnahme des Prüfgutes und mindestens einen elektrischen Wechselsignalgenerator zum Anlegen eines elektrischen Wechselsignals mit einer bestimmten Grundfrequenz und einer bestimmten Signalform an den Messkondensator. Die kapazitive Messschaltung ist vom mindestens einen Wechselsignalgenerator derart abgekoppelt, dass sie die Grundfrequenz und die Signalform des angelegten Wechselsignals nicht wesentlich beeinflusst. Die Vorrichtung beinhaltet ferner Detektionsmittel zur Detektion mindestens einer elektrischen Messgrösse eines am Messkondensator abgegriffenen elektrischen Signals. Der mindestens eine Wechselsignalgenerator ist dazu eingerichtet, die Grundfrequenz und/oder die Signalform des angelegten Wechselsignals zu verändern.

Im erfindungsgemäßen Verfahren zur kapazitiven Untersuchung eines bewegten länglichen, textilen Prüfgutes wie Kardenband, Vorgarn, Garn oder Gewebe wird das Prüfgut in einen Messkondensator eingebracht. Ein elektrisches Wechselsignal mit einer bestimmten Grundfrequenz und einer bestimmten Signal form wird von mindestens einem Wechselsignalgenerator erzeugt und an den Messkondensator angelegt. Der Messkondensator wird vom mindestens einen Wechselsignalgenerator derart abgekoppelt, dass er die Grundfrequenz und die Signalform des angelegten Wechselsignals nicht wesentlich beeinflusst. Mindestens eine elektrische Messgrösse eines am Messkondensator angelegten elektrischen Signals wird detektiert. Die Grundfrequenz und/oder die Signalform des angelegten Wechselsignals werden verändert.

Dank der Erfindung kann das an der Kondensatoranordnung angelegte Wechselsignal bezüglich seiner Grundfrequenz und/oder Signalform auf einfache Weise und mit hoher Genauigkeit eingestellt werden. Dadurch kann es optimal an das jeweilige Prüfgut, die gewählte Messmethode und/oder die herrschenden Umgebungsbedingungen angepasst werden. Die Erfindung eröffnet sogar Möglichkeiten für gänzlich neue Messmethoden wie z. B. Messungen mit Frequenz- oder Phasenmodulation, sich verändernde gegenseitige Phasenlagen etc. Aus Messungen mit mindestens zwei Wechselsignalen mit definierten gegenseitigen Phasenlagen lassen sich entsprechend mehrere Parameter des Prüfgutes ermitteln, z. B. die Masse, der Feuchtegehalt und die Materialzusammensetzung (bzw. der Anteil an Fremdstoffen) des Prüfgutes. Verfahren zur Auswertung von Messsignalen aus derartigen Messungen sind z. B. aus der EP-0'924'513 A1 und der WO-2007/115416 A1 bekannt.

### AUFZÄHLUNG DER ZEICHNUNGEN

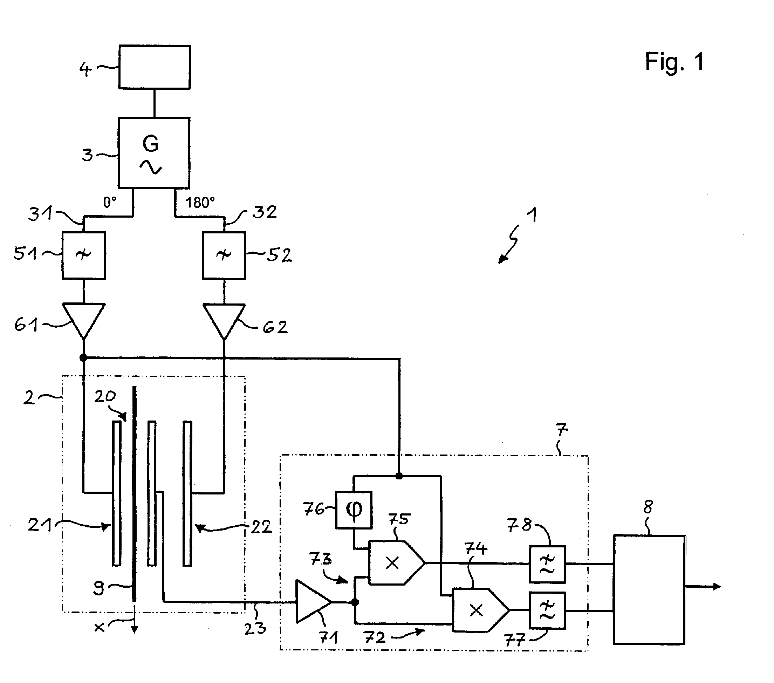

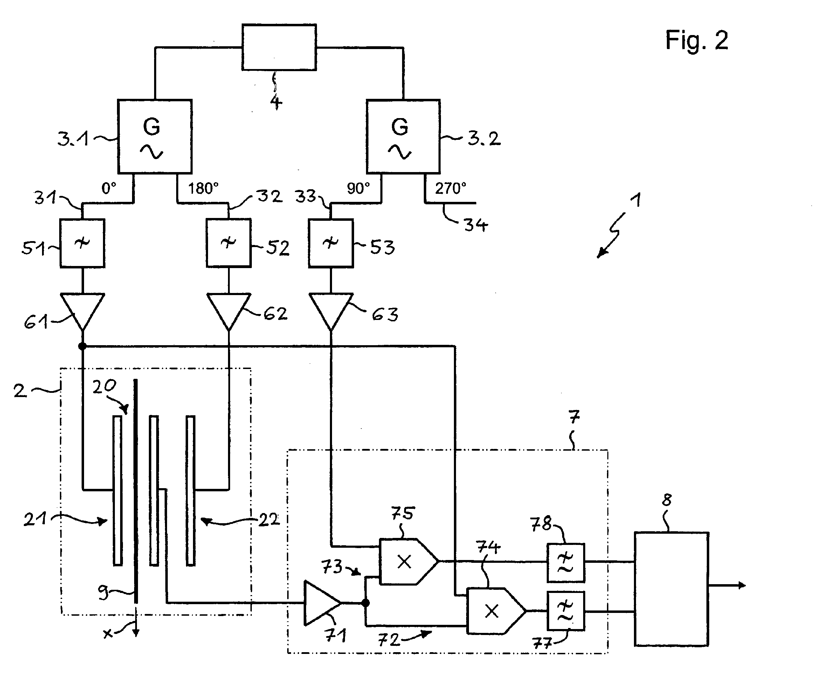

Nachfolgend wird die Erfindung anhand der schematischen Zeichnung detailliert erläutert. Figuren 1 und 2 zeigen Schaltschemata für zwei Ausführungsformen der erfindungsgemässen Vorrichtung.

### AUSFÜHRUNG DER ERFINDUNG

Die nachfolgend diskutierten bevorzugten Ausführungsformen der Erfindung verwenden mindestens einen Synthesizer als Wechselsignalgenerator. Dies soll nicht etwa einschränkend verstanden werden. Selbstverständlich können in der Erfindung viele andere Wechselsignalgeneratoren zum Einsatz kommen, die dem Fachmann an sich bekannt sind.

In Figur 1 ist ein elektrisches Schaltschema einer ersten Ausführungsform der erfindungsgemässen Vorrichtung 1 dargestellt. Die Vorrichtung 1 beinhaltet eine Messschaltung 2 mit einem Messkondensator 21 für ein entlang seiner Längsrichtung x bewegtes längliches Prüfgut 9, bspw. Garn. Der Messkondensator 21 weist zwei parallele Kondensatorplatten und eine dazwischen liegende Durchgangsöffnung 20 für das Prüfgut 9 auf. Fakultativ ist in der Messschaltung 2 ein Referenzkondensator 22 zur Erhöhung der Messgenauigkeit und zur Ausschaltung oder Verringerung von unerwünschten Umgebungseinflüssen wie Luftfeuchtigkeit oder Lufttemperatur vorhanden. Der Referenzkondensator 22 ist vorzugsweise gleich aufgebaut wie der Messkondensator 21, mit dem Unterschied, dass er nicht vom Prüfgut 9 durchlaufen wird. Der Messkondensator 21 und der Referenzkondensator 22 sind in Serie zueinander geschaltet und bilden zusammen einen kapazitiven Spannungsteiler. Selbstverständlich kann die Messschaltung 2 noch weitere, hier nicht eingezeichnete Komponenten beinhalten.

Die Vorrichtung 1 beinhaltet ferner einen z. B. als Synthesizer ausgebildeten elektrischen Wechselspannungsgenerator 3 zur Erzeugung eines Wechselspannungssignals, welches an die Messschaltung 2 angelegt wird. Dadurch wird die Durchgangsöffnung 20 des Messkondensators 21 von einem elektrischen Wechselfeld beaufschlagt, welches mit dem Prüfgut 9 wechselwirkt. Demzufolge kann aus einem Ausgangssignal des Messkondensators 21 auf Eigenschaften des Prüfgutes 9 geschlossen werden. Auch der Referenzkondensator 22 wird mit einem elektrischen Wechselfeld beaufschlagt.

In einer bevorzugten Ausführungsform wird als Synthesizer 3 ein direkter digitaler Synthesizer (direct digital synthesizer, DDS) eingesetzt. Der DDS kann von einer digitalen Schnittstelle 4 angesteuert werden.

In Figur 1 ist ein Ausführungsbeispiel dargestellt, in welchem der Synthesizer 3 ein Wechselspannungssignal, bspw. ein sinusförmiges Signal einer bestimmten Frequenz erzeugt, wobei auch Signalkomponenten mit anderen als der Grundfrequenz auftreten können. Zur Untersuchung von Garn 9 und anderen länglichen textilen Gebilde eignen sich besonders Frequenzen aus dem Bereich zwischen 1 MHz und 100 MHz, vorzugsweise zwischen 5 MHz und 50 MHz und bspw. ungefähr gleich 10 MHz. Der Synthesizer 3 weist vorzugsweise zwei Ausgangsleitungen 31, 32 auf, wobei auf einer ersten Ausgangsleitung 31 ein erstes Signal und auf einer zweiten Ausgangsleitung 32 ein zweites Signal ausgegeben wird, das im Wesentlichen mit dem ersten Signal identisch, jedoch bezüglich desselben um 180° phasenverschoben ist.

Die beiden vom Synthesizer 3 erzeugten Signale können gegebenenfalls von entsprechenden Filtern 51, 52 gefiltert werden. Danach werden sie von jeweils einem Verstärker 61, 62, z. B. einem Operationsverstärker, verstärkt. Die so verstärkten Signale werden in die Messschaltung 2 eingespiesen. Die Messschaltung 2 erhält somit zwar vom Synthesizer 3 erzeugte Wechselspannungssignale, ist aber vom Synthesizer 3 durch die Verstärker 61, 62 derart abgekoppelt, dass sie Parameter der vom Synthesizer 3 erzeugten Wechselspannungssignale nicht beeinflusst.

Der Messschaltung 2 ist vorzugsweise ein Demodulator 7 für ein auf einer elektrischen Leitung 23 ankommendes Ausgangssignal der Messschaltung 2 nachgeschaltet. Der Demodulator 7 dient der Demodulation des analogen Ausgangssignals der Messschaltung 2, d. h. der analogen Aufbereitung und der Extraktion eines niederfrequenten Signals aus dem an der Messschaltung 2 abgegriffenen elektrischen Signal. Im Ausführungsbeispiel von Fig. 1 beinhaltet der Demodulator 7 zunächst einen Verstärker 71 zur Verstärkung des Ausgangssignals. Das Ausgangssignal des Verstärkers 71 wird auf zwei Teilpfade 72, 73 aufgeteilt und bei zwei verschiedenen Phasen demoduliert. Die Demodulation wird im Wesentlichen synchron, als eine Multiplikation der Teilsignalkomponenten mit dem am Messkondensator 21 angelegten Wechselspannungssignal mittels Multiplizierer 74, 75 ausgeführt. Die Phasenverschiebung in einem Teilpfad 73 wird mit einem Phasenschieber 76 eingeführt. Sie beträgt vorzugsweise 90°, um ein Quadratursignal zu erhalten. Es ist aber durchaus möglich, eine andere Phasenschiebung zu wählen. Zur Glättung werden beide Signale durch je einen Tiefpassfilter 77, 78 geschickt und danach einer Auswerteeinheit 8 zugeführt. Die Auswerteeinheit 8 kann eine analoge elektrische Schaltung oder eine digitale Schaltung mit einem Prozessor beinhalten.

Schon das Ausführungsbeispiel von Figur 1, in dem der Synthesizer 3 ein monofrequentes Wechselspannungssignal liefert, hat grosse Vorteile gegenüber herkömmlichen Vorrichtungen, in denen die Messschaltung ein Teil der Wechselspannungsquelle ist. Die Frequenz des Wechselspannungssignals kann innerhalb eines sehr grossen Bereichs, der durch den Synthesizer 3 selbst beschränkt ist, frei und mit grosser Genauigkeit eingestellt werden.

Die Erfindung bietet aber noch viel mehr Freiheiten bezüglich des Wechselspannungssignals. Das vom Synthesizer 3 an die Messschaltung 2 abgegebene Signal kann mehrere Signalkomponenten mit unterschiedlichen Frequenzen beinhalten. So kann es vorteilhaft sein, nebst einer hochfrequenten Komponente im MHz-Bereich eine tieffrequente Komponente zur Verfügung zu stellen mit einer Frequenz aus dem Bereich zwischen 10 kHz und 1000 kHz, vorzugsweise zwischen 50 kHz und 500 kHz und bspw. ungefähr gleich 200 kHz. Es ist auch nicht nötig, sinusförmige Signalkomponenten zu verwenden, wobei jedoch bekanntlich jedes periodische Signal durch Fourier-Entwicklung in sinusförmige Komponenten zerlegbar ist.

Zusammenfassend kann also der Synthesizer 3 eine beliebige Anzahl von Signalkomponenten zu einem Wechselspannungssignal mischen, wobei die Signalformen, Frequenzen, Amplituden und gegenseitigen Phasenlagen der verschiedenen Komponenten beliebig wählbar sind.

Figur 2 zeigt ein Schaltschema einer zweiten Ausführungsform für die erfindungsgemässe Vorrichtung 1. Hier werden zwei monofrequente Wechselspannungssignale erzeugt, die gegeneinander um eine vorbestimmte Phasendifferenz verschoben sind. Dies wird mit zwei Synthesizern 3.1, 3.2, vorzugsweise DDS, erreicht, die mit Vorteil von einer einzigen digitalen Schnittstelle 4 angesteuert werden. Die digitale Schnittstelle 4 koordiniert die beiden Synthesizer 3.1, 3.2 derart, dass die gewünschte Phasenverschiebung zustande kommt. Das phasenverschobene Signal des zweiten Synthesizers 3.2 wird auf einer Ausgangsleitung 33 ausgegeben und zur Demodulation des Ausgangssignals der Messschaltung 2 verwendet, analog zur Ausführungsform von Figur 1, wo zu diesem Zweck der Phasenschieber 76 im Demodulator 7 eingesetzt wurde (vgl. Figur 1). Die Phasenverschiebung beträgt vorzugsweise 90°, wodurch ein Quadratursignal zur Verfügung gestellt wird. Sie könnte aber auch andere Werte annehmen oder verändert werden. Zur Filterung und Verstärkung des pahsenverschobenen Signals des zweiten Synthesizers 3.2 können, analog zum ersten Synthesizer 3.1, ein Filter 53 bzw. ein Verstärker 63 eingesetzt werden. Im Ausführungsbeispiel von Figur 2 bleibt der zweite Ausgang 34 des zweiten Synthesizers 3.2, der gegenüber dem ersten Ausgang 33 um 180° und somit gegenüber dem ersten Ausgang 31 des ersten Synthesizers 3.1 um 270° verschoben ist, ungenutzt. Er könnte jedoch auch zur Auswertung des Ausgangssignals der Messschaltung 2 eingesetzt werden. Alternativ zur Ausführungsform mit zwei Synthesizern 3.1, 3.2 könnten die verschiedenen phasenverschobenen Signale auch von einem einzigen Synthesizer mit entsprechenden Ausgängen zur Verfügung gestellt werden.

Es ist auch möglich, mehr als zwei phasenverschobene Signale zur Auswertung zu verwenden, die von einem einzigen oder von mehreren Synthesizern zur Verfügung gestellt werden. Damit lassen sich mehrere Eigenschaften des Prüfgutes 9 detektieren, z. B. die Prüfgutmasse, die im Prüfgüt 9 enthaltene Feuchtigkeit und allenfalls im Prüfgut 9 enthaltene Fremdstoffe wie Polypropylen. Darüber hinaus kann mit vielen solchen Signalen das Mischungsverhältnis verschiedener Rohbaumwollarten, aus denen das Prüfgut zusammengesetzt ist, ermittelt werden.

Selbstverständlich ist die vorliegende Erfindung nicht auf die oben diskutierten Ausführungsformen beschränkt. Bei Kenntnis der Erfindung wird der Fachmann weitere Varianten herleiten können, die auch zum Gegenstand der vorliegenden Erfindung gehören. Insbesondere können verschiedene an sich bekannte elektrische Wechselsignalgeneratoren zum Einsatz kommen.

### BEZUGSZEICHENLISTE

- 1: Vorrichtung
- 2: Messschaltung
- 20: Durchgangsöffnung
- 21: Messkondensator
- 22: Referenzkondensator

- 3: Wechselsignalgenerator
- 31, 32: Ausgangsleitungen des Wechselsignalgenerators
- 33, 34: Ausgangsleitungen eines zweiten Wechselsignalgenerators

- 4: digitale Schnittstelle

- 51-53: Filter

- 61-63: Verstärker

- 7: Demodulator
- 71: Verstärker
- 72, 73: Teilpfade des Demodulators
- 74, 75: Multiplizierer
- 76: Phasenschieber
- 77, 78: Tiefpassfilter

- 8: Auswerteschaltung

- 9: Prüfgut

- x: Längrichtung des Prüfgutes

## Patentansprüche

1. Vorrichtung (1) zur kapazitiven Untersuchung eines bewegten länglichen textilen Prüfgutes (9) wie Kardenband, Vorgarn, Garn oder Gewebe, beinhaltend eine kapazitive Messschaltung (2) mit einem Messkondensator (21) zur Aufnahme des Prüfgutes (9),
mindestens einen elektrischen Wechselsignalgenerator (3, 3.1, 3.2) zum Anlegen eines elektrischen Wechselsignals mit einer bestimmten Grundfrequenz und einer bestimmten Signalform an den Messkondensator (21), wobei
die kapazitive Messschaltung (2) vom mindestens einen Wechselsignalgenerator (3, 3.1, 3.2) derart abgekoppelt ist, dass sie die Grundfrequenz und die Signalform des angelegten Wechselsignals nicht wesentlich beeinflusst, und
Detektionsmittel (7) zur Detektion mindestens einer elektrischen Messgrösse eines am Messkondensator (21) abgegriffenen elektrischen Signals,
**dadurch gekennzeichnet, dass**
der mindestens eine Wechselsignalgenerator (3, 3.1, 3.2) zur Veränderung der Grundfrequenz und/oder der Signalform des angelegten Wechselsignals eingerichtet ist.

2. Vorrichtung (1) nach Anspruch 1, wobei der mindestens eine Wechselsignalgenerator (3, 3.1, 3.2) derart eingerichtet ist, dass zusätzlich eine Phasenlage und/oder eine Amplitude des von ihm erzeugten Wechselsignals vorgebbar sind.

3. Vorrichtung (1) nach einem der vorangehenden Ansprüche, wobei dem mindestens einen Wechselsignalgenerator (3, 3.1, 3.2) mindestens ein Filter (51-53) zur Filterung des vom Wechselsignalgenerator (3, 3.1, 3.2) erzeugten Wechselsignals nachgeschaltet ist.

4. Vorrichtung (1) nach einem der vorangehenden Ansprüche, wobei der mindestens eine Wechselsignalgenerator (3, 3.1, 3.2) aus der folgenden Menge ausgewählt ist: RC-Oszillator, LC-Oszillator, Quarz-Oszillator, Oszillator mit Keramikresonator, Oszillator mit SAW-Komponente, Oszillator mit Logikbausteinen, Synthesizer, Phasenregelschleife, Pulsweitenmodulator, Kippstufe.

5. Vorrichtung (1) nach Anspruch 4, wobei der mindestens eine Wechselsignalgenerator (3, 3.1, 3.2) ein direkter digitaler Synthesizer ist.

6. Vorrichtung (1) nach einem der vorangehenden Ansprüche, wobei mehrere Wechselsignalgeneratoren (3.1, 3.2) vorhanden sind, und Steuermittel (4) zur Ansteuerung der Wechselsignalgeneratoren (3.1, 3,2) derart ausgebildet sind, dass Phasen von Wechselsignalen verschiedener Wechselsignalgeneratoren (3.1, 3.2) um vorgegebene Phasendifferenzen gegeneinander phasenverschiebbar sind.

7. Vorrichtung (1) nach einem der vorangehenden Ansprüche, wobei die Detekionsmittel (7) einen der Kondensatoranordnung (21) nachgeschalteten Demodulator mit einem Multiplizierer (74) zur Multiplikation des an der Kondensatoranordnung (21) abgegriffenen elektrischen Signals mit einem Wechselsignal mindestens eines Wechselsignalgenerators (3, 3.2) beinhalten.

8. Verfahren zur kapazitiven Untersuchung eines bewegten länglichen textilen Prüfgutes (9) wie Kardenband, Vorgarn, Garn oder Gewebe, wobei das Prüfgut (9) in einen Messkondensator (21) eingebracht wird,
ein elektrisches Wechselsignal mit einer bestimmten Grundfrequenz und einer bestimmten Signalform von mindestens einem Wechselsignalgenerator (3, 3.1, 3.2) erzeugt und an den Messkondensator (21) angelegt wird,
der Messkondensator (21) vom mindestens einen Wechselsignalgenerator (3, 3.1, 3.2) derart abgekoppelt wird, dass er die Grundfrequenz und die Signalform des angelegten Wechselsignals nicht wesentlich beeinflusst, und
mindestens eine elektrische Messgrösse eines am Messkondensator (21) abgegriffenen elektrischen Signals detektiert wird,
**dadurch gekennzeichnet, dass**
die Grundfrequenz und/oder die Signalform des angelegten Wechselsignals verändert werden.

9. Verfahren nach Anspruch 8, wobei zusätzlich eine Phasenlage und/oder eine Amplitude des Wechselsignals verändert werden.

10. Verfahren nach Anspruch 8 oder 9, wobei mehrere Wechselsignale erzeugt werden, deren gegenseitige Phasen um vorgegebene Phasendifferenzen gegeneinander phasenverschoben sind.

11. Verfahren nach einem der Ansprüche 8-10, wobei zur Detektion der mindestens einen elektrischen Messgrösse das an der Kondensatoranordnung (21) abgegriffene elektrische Signal demoduliert wird, indem es mit einem Wechselsignal multipliziert wird.

## Claims

1. An apparatus (1) for the capacitive examination of a moved elongated test subject (9), containing
a capacitive measuring circuit (2) with a measuring capacitor (21) for receiving the test subject (9),
at least one electric alternating signal generator (3, 3.1, 3.2) for applying an electric alternating signal with a specific basic frequency and a specific signal shape to the measuring capacitor (21), with
the capacitive measuring circuit (2) being uncoupled from the at least one alternating signal generator (3, 3.1, 3.2) in such a way that it does not relevantly influence the basic frequency and the signal shape of the applied alternating signal, and
detection means (7) for detecting at least one electric measuring variable of an electric signal tapped from the capacitor arrangement (21),
**characterized in that**
the at least one alternating signal generator (3, 3.1, 3.2) is arranged for changing the basic frequency and/or the signal shape of the applied alternating signal.

2. The apparatus (1) according to claim 1, wherein the at least one alternating signal generator (3, 3.1, 3.2) is arranged in such a way that in addition a phase position and/or an amplitude of the alternating signal generated thereby are predeterminable.

3. The apparatus (1) according to one of the preceding claims, wherein at least one filter (51 to 53) for filtering the alternating signal generated by the alternating signal generator (3, 3.1, 3.2) is connected in series with the at least one alternating signal generator (3, 3.1, 3.2).

4. The apparatus (1) according to one of the preceding claims, wherein the at least one alternating signal generator (3, 3.1, 3.2) is chosen from the following group: RC oscillator, LC oscillator, quartz oscillator, oscillator with ceramic resonator, oscillator with SAW component, oscillator with logic units, synthesizer, phase-locked loop, pulse-width modulator, trigger circuit.

5. The apparatus (1) according to claim 4, wherein the at least one alternating signal generator (3, 3.1, 3.2) is a direct digital synthesizer.

6. The apparatus (1) according to one of the preceding claims, wherein several alternating signal generators (3.1, 3.2) are present, and control means (4) for controlling the alternating signal generators (3.1, 3.2) are arranged in such a way that phases of alternating signals of different alternating signal generators (3.1, 3.2) are phase-shiftable against one another by predetermined phase differences.

7. The apparatus (1) according to one of the preceding claims, wherein the detection means (7) contain a demodulator with a multiplier (74) connected in series with the capacitor arrangement (21) for multiplying the electric signal tapped from the capacitor arrangement (21) with an alternating signal of at least one alternating signal generator (3, 3.2).

8. A method for the capacitive examination of a moved elongated test subject (9),
wherein
the test subject (9) is introduced into a measuring capacitor (21),
an electric alternating signal with a specific basic frequency and a specific signal shape is generated by at least one alternating signal generator (3, 3.1, 3.2) and is applied to the measuring capacitor (21),
the measuring capacitor (21) is uncoupled from the at least one alternating signal generator (3, 3.1, 3.2) in such a way that it does not relevantly influence the basic frequency and the signal shape of the applied alternating signal, and
at least one electric measuring variable of an electric signal tapped from the measuring capacitor (21) is detected,
**characterized in that**
the basic frequency and/or the signal shape of the applied alternating signal are changed.

9. The method according to claim 8, wherein additionally a phase position and/or an amplitude of the alternating signal are changed.

10. The method according claim 8 or 9, wherein several alternating signals are generated whose mutual phases are phase-shifted against one another by predetermined phase differences.

11. The method according to one of the claims 8 to 10, wherein the electrical signal tapped from the capacitor arrangement (21) is demodulated for detecting the at least one electric measuring variable, in that it is multiplied with an alternating signal.

## Revendications

1. Dispositif (1) pour l'analyse capacitive d'un échantillon textile (9) allongé en mouvement tel qu'un fil de carde, un fil mèche, un filé ou un tissu, contenant un circuit de mesure capacitif (2) avec un condensateur de mesure (21) pour capter l'échantillon (9),
au moins un générateur de signaux alternatifs électrique (3, 3.1, 3.2) destiné à appliquer un courant alternatif d'une certaine fréquence fondamentale et d'une certaine forme de signal au condensateur de mesure (21),
le circuit de mesure capacitif (2) étant découplé d'au moins un générateur de signaux alternatifs électrique (3, 3.1, 3.2) de telle manière qu'il n'influence pas notablement la fréquence fondamentale ni la forme de signal du signal alternatif appliqué, et
des moyens de détection (7) pour la détection d'au moins une grandeur de mesure électrique d'un signal électrique capté sur le condensateur de mesure (21),
**caractérisé en ce que**
l'au moins un générateur de signaux alternatifs électrique (3, 3.1, 3.2) est conçu pour modifier la fréquence fondamentale et/ou la forme de signal du signal alternatif appliqué.

2. Dispositif (1) selon la revendication 1, dans lequel l'au moins un générateur de signaux alternatifs électrique (3, 3.1, 3.2) est conçu de telle manière qu'une position de phase et/ou une amplitude du signal alternatif qu'il génère puissent en outre être prédéfinies.

3. Dispositif (1) selon l'une des revendications précédentes, dans lequel est monté en aval de l'au moins un générateur de signaux alternatifs électrique (3, 3.1, 3.2) au moins un filtre (51-53) destiné à filtrer le signal alternatif généré par le générateur de signaux alternatifs électrique (3, 3.1, 3.2).

4. Dispositif (1) selon l'une des revendications précédentes, dans lequel l'au moins un générateur de signaux alternatifs électrique (5, 3.1, 3.2) est choisi parmi le groupe suivant : oscillateur RC, oscillateur LC, oscillateur à quartz, oscillateur à résonateur en céramique, oscillateur avec composant SAW, oscillateur à composants logiques, synthétiseurs, boucle de régulation de phase, modulateur d'amplitude, circuit de base de temps.

5. Dispositif (1) selon la revendication 4, dans lequel l'au moins un générateur de signaux alternatifs électrique (3, 3.1, 3.2) est un synthétiseur numérique direct.

6. Dispositif (1) selon l'une des revendications précédentes, dans lequel plusieurs générateurs de signaux alternatifs électriques (3.1, 3.2) sont prévus et des moyens de commande (4) pour la commande des générateurs de signaux alternatifs électriques (3.1, 3.2) sont conformés de telle manière que les phases des signaux alternatifs de générateurs de signaux alternatifs électriques (3.1, 3.2) différents puissent être décalées en phase d'une différence de phase prédéterminée les unes par rapport aux autres.

7. Dispositif (1) selon l'une des revendications précédentes, dans lequel les moyens de détection (7) comprennent un démodulateur monté en aval de la disposition de condensateurs (21) avec un multiplicateur (74) pour multiplier le signal capté sur la disposition de condensateurs (21) par un signal alternatif d'au moins un générateur de signaux alternatifs électrique (3, 3.2).

8. Procédé pour l'analyse capacitive d'un échantillon textile allongé (9) en mouvement tel qu'un fil de carde, un fil mèche, un filé ou un tissu, dans lequel
l'échantillon (9) est introduit dans un condensateur de mesure (21),
un signal alternatif électrique d'une certaine fréquence fondamentale et d'une certaine forme de signal est généré par au moins un générateur de signaux alternatifs électrique (3, 3.1, 3.2) et appliqué au condensateur de mesure (21),
le condensateur de mesure (21) est découplé d'au moins un générateur de signaux alternatifs électrique (3, 3.1, 3.2) de telle manière qu'il n'influence pas notablement la fréquence fondamentale ni la forme de signal du signal alternatif appliqué, et
au moins une grandeur de mesure électrique d'un signal électrique capté au niveau du condensateur de mesure (21) est détectée,
**caractérisé en ce que**
la fréquence fondamentale et/ou la forme de signal du signal alternatif appliqué sont modifiées.

9. Procédé selon la revendication 8, dans lequel une position de phase et/ou une amplitude du signal alternatif sont en outre modifiées.

10. Procédé selon la revendication 8 ou 9, dans lequel plusieurs signaux alternatifs sont générés, dont les phases mutuelles sont décalées les unes par rapport aux autres d'une différence de phase prédéterminée.

11. Procédé selon l'une des revendications 8 à 10, dans lequel, en vue de la détection de l'au moins une grandeur de mesure électrique, le signal électrique capté sur la disposition de condensateurs (21) est démodulé en étant multiplié par un signal alternatif.
